# EUROPEAN PATENT SPECIFICATION

(11) **EP 1 007 963 B1**
(45) Date of publication and mention of the grant of the patent: **23.10.2002**
(21) Application number: 98944154.8
(22) Date of filing: 20.08.1998
(51) Int. Cl.: G01N 33/50, C12Q 1/68

(54) **METHODS AND REAGENTS FOR IDENTIFYING MODULATORS OF NEURONAL APOPTOSIS**
METHODE UND REAGENZIEN ZUM IDENTIFIZIEREN VON MODULATOREN NEURONALER APOPTOSE
PROCEDES ET REACTIFS PERMETTANT D'IDENTIFIER LES MODULATEURS DE L'APOPTOSE NEURONALE

(30) Priority: 25.08.1997 US 917662
(43) Date of publication of application: 14.06.2000
(73) Proprietor: McGILL UNIVERSITY, Montreal, Québéc H3A 2T5 (CA)
(72) Inventor: MILLER, Freda, Diane, Montreal, Quebec H4A 3B3 (CA); ALOYZ, Raquel, Silvia, Montreal, Quebec H2K 2K1 (CA); KAPLAN, David, Montreal, Quebec H4A 3B3 (CA)
(74) Representative: Grund, Martin, Dr.
(86) International application number: IB9801525
(87) International publication number: WO99010741

(56) References cited:
- WO-A-95/19367
- WO-A-95/28497
- WO-A-97/10349
- WO-A-98/22608
- SLACK R S ET AL: "ADENOVIRUS-MEDIATED GENE TRANSFER OF THE TUMOR SUPPRESSOR, P53, INDUCES APOPTOSIS IN POSTMITOTIC NEURONS" THE JOURNAL OF CELL BIOLOGY, vol. 135, no. 4, November 1996, pages 1085-1096, XP002061111 cited in the application
- KOH, J-Y ET AL: "STAUROSPORINE-INDUCED NEURONAL APOPTOSIS" EXPERIMENTAL NEUROLOGY, vol. 135, no. 2, 1995, pages 153-159, XP002092237 cited in the application
- ALOYZ, R. ET AL: "Neurotrophin withdrawal-induced intracellular mechanisms mediating apoptosis of sympathetic neurons." SOCIETY FOR NEUROSCIENCE ABSTRACTS, (1997) VOL. 23, NO. 1-2, PP. 1986. MEETING INFO.: 27TH ANNUAL MEETING OF THE SOCIETY FOR NEUROSCIENCE NEW ORLEANS, LOUISIANA, USA OCTOBER 25-30, 1997 ISSN: 0190-5295., XP002092238
- Freemann RS et al, Neuron 1994, Feb.; 12(2):343-55. Medline Abstract (PMID: 8110463)
- Sakhi S. et al, Proc Natl Acad Sci USA, 1994, Aug 2; 91(16): 7525-9. Medline Abstract (PMID: 8052613)

## Description

### FIELD OF THE INVENTION

The invention relates to neuronal apoptosis.

### BACKGROUND OF THE INVENTION

The development of strategies to promote repair of the degenerating or traumatized nervous system is a major ongoing therapeutic challenge. Current strategies to treat the injured nervous system include transplantation surgery and treatments utilizing recombinant endogenous proteins, such as growth factors. Both of these therapies have significant limitations; the surgical approaches are expensive, invasive and labor-intensive, while growth factors generally cannot cross the blood-brain barrier and often have pleiotropic biological activities.

An alternative approach to the problem of nerve trauma and regeneration is the development of small molecule therapeutics able to cross the blood-brain barrier and promote the survival or repair of traumatized neurons. However, one of the fundamental technological problems associated with the identification of therapeutically relevant small molecules is the lack of suitable high throughput screens for compounds effective on primary neurons. This lack of suitable screens derives from two major considerations. First, recent findings indicate that primary neurons differ significantly in their survival and growth signalling pathways from any of the transformed or immortalized cell lines that are currently available, making screens using cell lines unreliable. Second, postmitotic neurons are: 1) only available in relatively small amounts; 2) difficult to genetically manipulate; and 3) difficult to culture as a purified cell population.

Recent studies have shown that neuronal cell death resulting from degeneration or trauma occurs by the process of apoptosis, the biochemical hallmarks of which include cytolemmal membrane blebbing, cell soma shrinkage, chromatin condensation, and DNA laddering. Slack, R.S. et al, *J. Cell.Biol*., 135:1085 (1996) have shown that following overexpression of p53, apoptosis may be induced in postmitotic neurons, while p53 does not play a major role in naturally occurring neuronal cell death. A method for screening of candidate substances capable of protecting cells from apoptosis has been described in WO97/10349. Furthermore, WO95/19367 discloses a method for identifying an agent regulating the expression of a gene involved in apoptosis by measuring the expression of a reporter gene under control of a p53-responsive element or the bax promoter which is activated by a p53-responsive element. It would be highly desirable to have a means for identifying compounds that reduce neuronal apoptosis.

### SUMMARY OF THE INVENTION

The invention provides methods for identifying compounds for the prevention and treatment of neuronal cell death.

In the first aspect, the invention features a method of identifying a compound which modulates neuronal apoptosis. The method includes the steps of: exposing a neuron to a test compound; exposing the neuron to a stimulus which is capable of initiating apoptosis of a neuronal cell; and assaying for an alteration in the level of p53, p21, p27 or phosphorylated Rb polypeptides relative to a neuron not exposed to the test compound, a decrease in the level indicating a compound which reduces neuronal apoptosis, and an increase in said level indicating a compound which enhances neuronal apoptosis, with proviso that the method is not performed on the human body.

In the second aspect, the invention features a method of identifying a compound which modulates neuronal apoptosis, which includes the steps of: exposing said neuron to a test compound; exposing a neuron to a stimulus which is capable of initiating apoptosis of a neuronal cell; and assaying for an alteration in the level of p53, p21 or p27 gene expression relative to a neuron not exposed to said test compound, a decrease in the level indicating a compound which reduces neuronal apoptosis, and an increase in the level indicating a compound which enhances neuronal apoptosis, with proviso that the method is not performed on the human body.

In various embodiments of the above aspects of the invention the alteration in protein levels is assayed for by assaying for an alteration in the level of p53, p21 or p27 mRNA; the neuron is selected from the group consisting of: a sympathetic neuron, a cortical neuron, a motor neuron, and a hippocampal neuron; the neuron is from a transgenic animal; (e.g., a transgenic animal having a reporter gene construct or a loss of function mutation); the neuron is exposed to an adenovirus vector; the stimulus is selected from the group consisting of serum withdrawal, growth factor withdrawal (e.g., nerve growth factor or neurotrophin), staurosporine exposure, glutamine exposure, NMDA exposure, DNA damage, exposure to reactive oxygen species, exposure to physical trauma, and axotomy; the stimulus is increased expression of a protein that can stimulate apoptosis (e.g., p53); the neuron contains a reporter gene operably linked to a transcriptional control region from a gene encoding p53, p21 or p27 (e.g., β-galactosidase, and its engineered variants, green fluorescent protein, and its engineered variants, chloramphenicol acetyltransferase, placental alkaline phosphatase, and luciferase; the neuron contains a reporter gene operably linked to a transcriptional control region from the gene encoding Tα1-tubulin; the assay further includes testing for an decreased level of reporter gene product; the decrease in the level of polypeptide is at least a 30% decrease in the level of p53, a 30% decrease in the level of p21, a 30% decrease level of p27 or a 30% decrease in the level of phosphorylated pRb; the exposing to the test compound is before or after the exposure to the stimulus; the assay is a Western blot; the assay is an enzyme-linked immunosorbant assay (ELISA); the assay is a reporter gene assay; the assay is a quantitative reverse transcription/polymerase chain reaction; the assay is an assay which measures mRNA levels by nucleic acid hybridization; the assaying measures the levels of at least two of the said polypeptides; and the assaying measures at least two of the said gene expression levels.

By "apoptosis" is meant the process of cell death wherein a dying cell displays a set of well-characterized biochemical hallmarks which include cytolemmal membrane blebbing, cell soma shrinkage, chromatin condensation, and DNA laddering.

By "stimulus which is capable of inducing apoptosis" or "apoptotic stimulus" is meant any chemical or physical treatment which initiates apoptosis as defined above. For example, nerve growth factor withdrawal, hypoxia, exposure to staurosporine, and cerebral ischemia are stimuli capable of inducing apoptosis in neurons.

By "neuron" is meant a cell of ectodermal embryonic origin derived from any part of the nervous system of an animal. Neurons express well-characterized neuron-specific markers which include class III β-tubulin, MAP2, and neurofillament proteins. Neurons includes without limitation, hippocampal, cortical, midbrain dopaminergic, motor, sensory, and sympathetic neurons.

By "expose" is meant to allow contact between an animal, cell, lysate or extract derived from a cell, or molecule derived from a cell, and a test compound or apoptotic stimulus.

By "treat" is meant to submit or subject an animal, cell, lysate or extract derived from a cell, or molecule derived from a cell to a test compound or apoptotic stimulus.

By "test compound" is meant a chemical, be it naturally-occurring or artificially-derived, that is surveyed for its ability to modulate cell death, by employing one of the assay methods described herein. Test compounds may include, for example, peptides, polypeptides, synthesized organic molecules, naturally occurring organic molecules, nucleic acid molecules, and components thereof.

By "assaying" is meant analyzing the effect of a treatment, be it chemical or physical, administered to whole animals or cells derived therefrom. The material being analyzed may be an animal, a cell, a lysate or extract derived from a cell, or a molecule derived from a cell. The analysis may be, for example, for the purpose of detecting altered gene expression, altered RNA stability, altered protein stability, altered protein levels, or altered protein biological activity. The means for analyzing may include, for example, antibody labeling, immunoprecipitation, phosphorylation assays, and methods known to those skilled in the art for detecting nucleic acids.

By "modulating" is meant changing, either by decrease or increase.

By "a decrease" is meant a lowering in the level of: a) protein, or protein phosphorylation, as measured by ELISA; b) reporter gene activity, of at least 30%, as measured by reporter gene assay, for example, lacZ/β-galactosidase, green fluorescent protein, luciferase, etc.; c) mRNA, levels of at least 30%, as measured by PCR relative to an internal control, for example, a "housekeeping" gene product such as β-actin or glyceraldehyde 3-phosphate dehydrogenase (GAPDH). In all cases, the lowering is preferably by 30%, more preferably by 40%, and even more preferably by 70%.

By "an increase" is meant a rise in the level of: a) protein, or protein phosphorylation, measured by ELISA; b) reporter gene activity, as measured by reporter gene assay, for example, lacZ/β-galactosidase, green fluorescent protein, luciferase, etc.; c) mRNA, as measured by PCR relative to an internal control, for example, a "housekeeping" gene product such as β-actin or glyceraldehyde 3-phosphate dehydrogenase (GAPDH). Preferably, the increase is by 2-fold, more preferably 3-fold.

By "alteration in the level of gene expression" is meant a change in gene activity such that the amount of a product of the gene, i.e., mRNA or polypeptide, is increased or decreased, that the stability of the mRNA or the polypeptide is increased or decreased.

By "reporter gene" is meant any gene which encodes a product whose expression is detectable and/or quantitatable by immunological, chemical, biochemical or biological assays. A reporter gene product may, for example, have one of the following attributes, without restriction: fluorescence (e.g., green fluorescent protein), enzymatic activity (e.g., lacZ/β-galactosidase, luciferase, chloramphenicol acetyltransferase), toxicity (e.g., ricin A), or an ability to be specifically bound by a second molecule (e.g., biotin or a detectably labelled antibody). It is understood that any engineered variants of reporter genes, which are readily available to one skilled in the art, are also included, without restriction, in the forgoing definition.

By "operably linked" is meant that a gene and a regulatory sequence are connected in such a way as to permit expression of the gene product under the control of the regulatory sequence.

By a "transgene" is meant a nucleic acid sequence which is inserted by artifice into a cell and becomes a part of the genome of that cell and its progeny. Such a transgene may be partly or entirely heterologous to the cell.

By "transgenic animal" an animal comprising a transgene as described above.

By "protein" or "polypeptide" or "polypeptide fragment" is meant any chain of more than two amino acids, regardless of post-translational modification (e.g., glycosylation or phosphorylation), constituting all or part of a naturally-occurring polypeptide or peptide, or constituting a non-naturally occurring polypeptide or peptide.

### BRIEF DESCRIPTION OF THE DRAWINGS

Figure 1 is a graph showing that inhibition of p53 by adenoviral protein E1B55K leads to rescue of sympathetic neuron apoptosis as induced by NGF withdrawal. Increasing the concentration of the E1B55K adenoviral vector used to introduce E155K into the cells leads to increased neuronal survival.
Figure 2 is a Western blot analysis for the tumor suppressor protein p53 in lysates from cultured neonatal sympathetic neurons at various timepoints after nerve growth factor (NGF) withdrawal (lane 1, control maintained in NGF; lane 2, 4 hours; lane 3, 16 hours; lane 4, 24 hours; lane 5, 36 hours). The arrow indicates the p53-immunoreactive band.
Figure 3 is a Western blot analysis for p21 in lysates of cultured neonatal sympathetic neurons at various timepoints following NGF withdrawal (lane 1, control maintained in NGF; lane 2, 12 hours; lane 3, 24 hours; lane 4, 36 hours). The arrow indicates the p21-immunoreactive band.
Figure 4 is a Western blot analysis for p27 in lysates of cultured neonatal sympathetic neurons at various timepoints following NGF withdrawal (lane 1, control maintained in NGF; lane 2, 4 hours; lane 3, 16 hours; lane 4, 24 hours; lane 5, 36 hours). The arrow indicates the p27-immunoreactive band.
Figure 5 is a Western blot analysis for Bad in lysates of cultured neonatal sympathetic neurons at various timepoints following NGF withdrawal (lane 1, control maintained in NGF; lane 2, 4 hours; lane 3, 16 hours; lane 4, 24 hours; lane 5, 36 hours). The arrow indicates the Bad-immunoreactive band.
Figure 6 is a Western blot analysis for Bax in a lysate of cultured neonatal sympathetic neurons following NGF withdrawal (lane 2, control maintained in NGF; lane 1, 36 hours). The arrow indicates the Bax-immunoreactive band.
Figure 7 is a Western blot analysis for Bcl2 polypeptide in lysates of cultured neonatal sympathetic neurons at various timepoints following NGF withdrawal (lane 1, control maintained in NGF; lane 2, 4 hours; lane 3, 16 hours; lane 4, 24 hours). The arrow indicates the Bcl2-immunoreactive band.
Figure 8 is a Western blot analysis for pRb in lysates of cultured neonatal sympathetic neurons at various timepoints following NGF withdrawal ( lane 1, control maintained in NGF; lane 2, 4 hours; lane 3, 16 hours; lane 4, 24 hours; lane 6, 36 hours; lane 7, control maintained in NGF). Arrows indicate pRb-immunoreactive bands. There is a shift to a higher molecular weight in later timepoints (compare lanes 6 and 7).

### DETAILED DESCRIPTION OF THE INVENTION

We have discovered that the levels of certain proteins reproducibly increase in neurons undergoing apoptotic cell death. We refer to these proteins as the death marker proteins and they include p53, p21, p27 and phosphorylated Rb (pRB) Fluctuations of the levels of proteins can be employed to identify alterations in populations of neurons undergoing apoptotic cell death. Furthermore, detection of "death marker" protein fluctuations can be incorporated into a screen for compounds that are capable of reducing or inhibiting neuronal cell death, such as cell death resulting from neurodegenerative disease (e.g., Alzheimer's disease, Huntington's disease, Parkinson's disease, and amyotrophic lateral sclerosis) or resulting from trauma such as ischemic stroke or axotomy. In addition, the monitoring of the death markers also can be used to test the relative neurotoxicity of a compound; such compounds might be useful, for example, as fertilizers, pesticides, and pharmaceutical agents for the treatment of proliferative diseases of the nervous system.

We have documented changes in intracellular levels of proteins occurring around the time of commitment to apoptosis, as measured using an NGF withdrawal assay provided below. We have found that the death marker proteins have specific response profiles which are hallmarks of the irreversible commitment of neurons to undergo apoptosis. These specific, reproducible, death-induced biochemical fluctuations, which will be described below, can be exploited in useful high-throughput screening assays for compounds, particularly small molecules, that halt or speed the commitment to cell death in primary neurons.

### Biochemical Changes in Dying Neurons

Cultures of primary neurons are easily prepared for use in apoptosis assays. Methods for isolating neurons can be found, for example, in Slack, R. S., et al., *J. Cell Biol*., 135:1085, (1996), or Belliveau, D. J., et al., *J. Cell Biol*., 136:375 (1997). After establishment of a healthy neuron culture, neurons can be induced to undergo apoptosis by exposure to any one of many well-characterized stimuli. Such a death stimulus for cultured neurons includes, but is not limited to, NGF withdrawal, serum withdrawal, exposure to hypoxic conditions, or the addition of chemicals that induce apoptosis, such as staurosporine or glutamate.

Apoptosis can also be induced *in vivo* by a myriad of techniques. In whole animals, a death stimulus for neurons *in situ* includes, for example, axotomy or ischemic trauma. After a suitable amount of time, protein or mRNA can be isolated from the neurons or the intact neurons may be cultured by well known methods (see, for example, Ausubel et al., *Current Protocols in Molecular Biology,* John Wiley & Sons, New York, NY, 1997, and Belliveau et al., *J. Cell Biol.,* 136:375, 1997) and isolated materials may then be subjected to assays as will be described in the Examples below.

One experimental system which can be used to assay for changes in death marker proteins in neurons undergoing cell death employs primary sympathetic neurons that are cultured for 3-5 days in nerve growth factor (NGF) to maintain their survival, after which time NGF is withdrawn to induce apoptosis. Death of the neuron occurs within 48 hours and requires transcription. Interestingly, the commitment to death is a reversible process up until a "commitment point" of approximately 15-18 hours after NGF withdrawal. Prior to the commitment point, the neurons can be rescued by re-supplying them with NGF; subsequent to the commitment point, the neurons can no longer be rescued, and are committed to die. As a part of the invention, we have shown that death due to NGF withdrawal in this assay is an apoptotic event (see Example V, below).

Using the NGF withdrawal assay we observed specific changes in the levels of certain proteins (termed the "death marker proteins") involved in the regulation of cell cycle progression and in proteins known to induce cell death, in sympathetic neurons induced to die by NGF withdrawal. These proteins include p53, p21, p27, Bad, Bax, Bcl2, and phosphorylated pRb. Other proteins having these activities show no detectable alterations.

The specific alterations are provided below and Table 1 provides the preferred parameters for use of these markers in assays for neurological apoptosis modulators. Taken alone or together, changes in the death proteins provide a unique, reliable signature for neurons at different stages of apoptotic cell death and allow one to distinguish between reversible and irreversible commitment to apoptotic cell death.

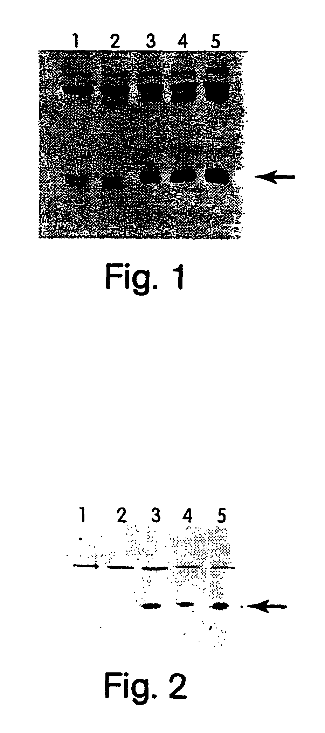

Levels of p53, a tumor suppressor protein that mediates cell cycle arrest, increase approximately 5 to 10-fold following NGF withdrawal (Figure 2). This increase commences between 12 and 16 hours after NGF withdrawal, and is then maintained. A 3-fold increase in p53 is sufficient to cause sympathetic neuron apoptosis.

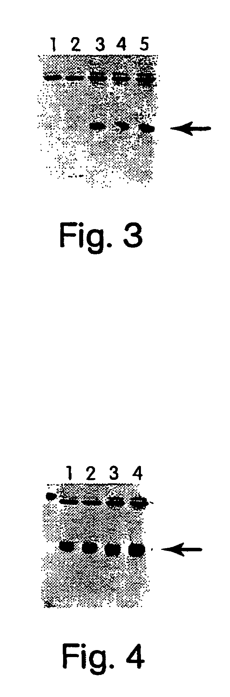

Levels of p21, a cyclin-dependent kinase inhibitor, increase between 12 and 24 hours, and remain elevated at 36 hours after NGF withdrawal (Figure 3).

Levels of p27, a cyclin-dependent kinase inhibitor involved in regulating cell cycle progression, increase approximately 3-fold, with a timecourse similar to that of p53 (Figure 4).

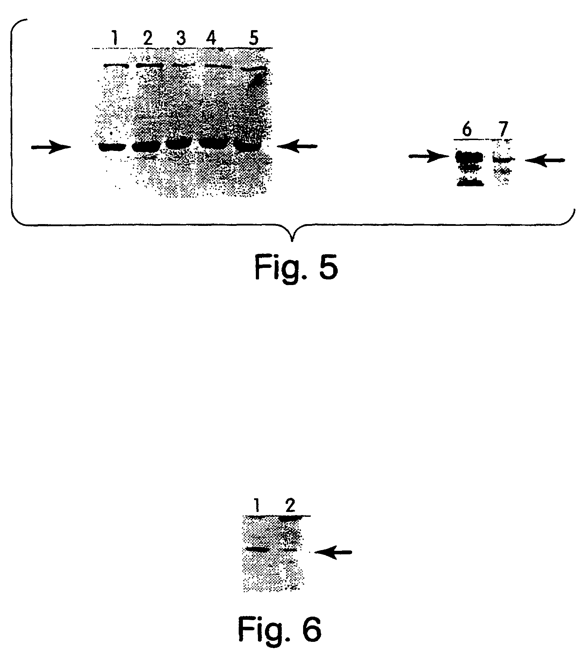

Levels of Bad, a protein that can induce apoptosis, increase 3 to 5 fold over 16 to 24 hours, a slower time course than that for p53 (Figure 5).

Levels of Bax, a protein that can induce apoptosis, and whose gene is a transcriptional target of p53, increased 36 hours after NGF withdrawal (Figure 6).

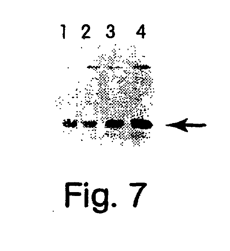

Levels of Bcl2, a protein that can inhibit apoptosis decrease slightly 12 or more hours after induction (Figure 7).

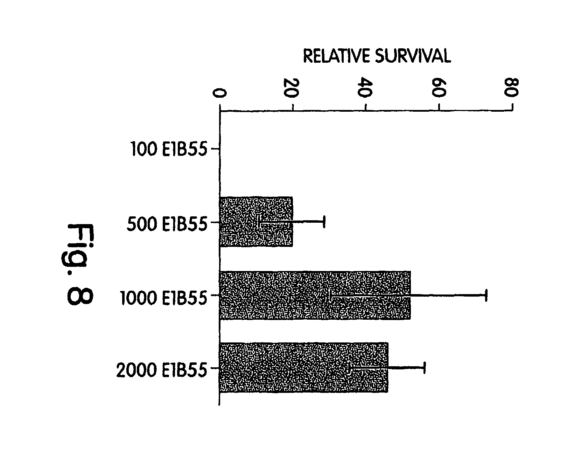

pRb, a tumor suppressor protein, becomes hyperphosphorylated by 16 hours after NGF withdrawal, and remains hyperphosphorylated at 36 hours post-NGF withdrawal (Figure 8).

The following table summarizes the changes in death marker proteins which may be used to measure commitment to apoptosis.

**TABLE I**

| Preferred Parameters for Measuring Death Markers | | |
|---|---|---|
| Marker for monitoring apoptosis | Inhibitor of Apoptosis* | Time period After Induction of Apoptosis |
| p53 | Any compound that reduces levels at least 30%, preferably 100% | 12 h to 36 h or more |
| | | |
| p21 | Any compound that reduces levels at least 30%, preferably 90% | 16 h to 36 h or more |
| | | |
| p27 | Any compound that reduces levels at least 30%, preferably 90% | 16 h to 36 h or more |
| | | |
| Bad | Any compound that reduces levels at least 20%, preferably 100% | 16 h to 36 h or more |
| | | |
| Bax | Any compound that reduces levels at least 20%, preferably 100% | 16 h to 36 h or more |
| | | |
| Bcl2 | Any compound that increases levels at least 20%, preferably 500% | 12 h to 36 h or more |
| | | |
| pRb | Any compound that reduces levels of the phosphorylated form at least 30%, preferably 100% | 12 h to 36 h or more |
| | | |
| | h = hours | |

| | | |
|---|---|---|
| * Relative to levels in a control not exposed to the test compound. | | |

To summarize, changes in intracellular levels of the death marker proteins can be employed to determine the cell death status of a neuron sample. Such diagnostic changes can be used in *in vivo* and *in vitro* assays to test compounds for their ability to modulate neuronal apoptotic cell death.

It is understood that variations of the assays described in Examples I-IV may be employed, such variations comprising, but not being limited to, the variations described below.

Other types of primary neurons, which are isolated by standard techniques, such as primary cortical neurons, hippocampal neurons, and motor neurons, also may be used. See, e.g., Brewer et al., *Nature,* 363:265-266, 1993 and Henderson et al., *Nature* 363:266-267, 1993.

Other methods of initiating neuronal killing, for example, withdrawal of serum or other neurotrophin growth factors such as BDNF; exposure to a hypoxic environment, exposure to chemicals known to induce apoptosis such as staurosporine or glutamate, may be employed See, e.g., Koh et al., *Exp. Neurol*., 135(2):153-159, 1995 and MacManus et al., *Exp. Cell Res.,* 233(2):310-320, 1997.

Cellular levels of other biochemical markers are employed as indication that neuronal death is modulated by test compounds. Measurement of a death protein polypeptide, mRNA, PCR products, and reporter gene activity of a reporter operatively linked to a death protein promoter are all a part of the invention. The assays can also employ methods of detecting modulation in levels of p53, p21, p27, pRb phosphorylation or any combination thereof.

The assays described herein can be used to test for compounds that decrease cell death and hence may have therapeutic value in the treatment of neurodegenerative disease and neurological trauma. The assays also can be used to screen compounds for neurotoxicity, such compounds being useful as pesticides or cancer therapeutics, for example.

### Secondary screens of test compounds that appear to modulate neuronal death.

After test compounds that appear to have neuronal death-modulating activity are identified, it may be necessary or desirable to subject these compounds to further testing. The invention provides such secondary confirmatory assays. For example, a compound that appears to inhibit cell death in early testing will be subject to additional assays to confirm that the levels of other cell death markers are reproducibly influenced by the compound. At late stages testing will be performed *in vivo* to confirm that the compounds initially identified to affect cell death in cultured neurons will have the predicted effect on *in vivo* neurons. In the first round of *in vivo* testing, neuronal cell death is initiated in animals, by well-known methods such as axotomy or cerebral ischemia, and then the compound is administered by one of the means described in the Therapy section immediately below. Neurons or neural tissue are isolated within hours to days following the insult, and are subjected to assays as described in the examples below.

### Test Compounds

In general, novel drugs for prevention or treatment of neuronal apoptosis are identified from large libraries of both natural product or synthetic (or semi-synthetic) extracts or chemical libraries according to methods known in the art. Those skilled in the field of drug discovery and development will understand that the precise source of test extracts or compounds is not critical to the screening procedure(s) of the invention. Accordingly, virtually any number of chemical extracts or compounds can be screened using the exemplary methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, NH) and Aldrich Chemical (Milwaukee, WI). Alternatively, libraries of natural compounds in the form of bacterial, fungal, plant, and animal extracts are commercially available from a number of sources, including Biotics (Sussex, UK), Xenova (Slough, UK), Harbor Branch Oceangraphics Institute (Ft. Pierce, FL), and PharmaMar, U.S.A. (Cambridge, MA). In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

In addition, those skilled in the art of drug discovery and development readily understand that methods for dereplication (e.g., taxonomic dereplication, biological dereplication, and chemical dereplication, or any combination thereof) or the elimination of replicates or repeats of materials already known for their therapeutic activities for neurodegenerative disorders should be employed whenever possible.

When a crude extract is found to prevent or delay neuronal apoptosis, further fractionation of the positive lead extract is necessary to isolate chemical constituents responsible for the observed effect. Thus, the goal of the extraction, fractionation, and purification process is the careful characterization and identification of a chemical entity within the crude extract having neuronal apoptosis- preventative or -palliative activities. The same assays described herein for the detection of activities in mixtures of compounds can be used to purify the active component and to test derivatives thereof. Methods of fractionation and purification of such heterogenous extracts are known in the art. If desired, compounds shown to be useful agents for treatment are chemically modified according to methods known in the art. Compounds identified as being of therapeutic value may be subsequently analyzed using a mammalian neuronal apoptosis model.

Below are examples of high-throughput systems useful for evaluating the efficacy of a molecule or compound in treating, preventing, or enhancing a neuronal apoptosis-associated condition.

### Therapy

Compounds identified using any of the methods disclosed herein, may be administered to patients or experimental animals with a pharmaceutically-acceptable diluent, carrier, or excipient, in unit dosage form. Conventional pharmaceutical practice may be employed to provide suitable formulations or compositions to administer such compositions to patients or experimental animals. Although intravenous administration is preferred, any appropriate route of administration may be employed, for example, parenteral, subcutaneous, intramuscular, intracranial, intraorbital, ophthalmic, intraventricular, intracapsular, intraspinal, intracisternal, intraperitoneal, intranasal, aerosol, or oral administration. Therapeutic formulations may be in the form of liquid solutions or suspensions; for oral administration, formulations may be in the form of tablets or capsules; and for intranasal formulations, in the form of powders, nasal drops, or aerosols.

Methods well known in the art for making formulations are found in, for example, "Remington's Pharmaceutical Sciences." Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for antagonists or agonists of the invention include ethylene-vinyl acetate copolymer particles, osmotic pumps, implantable infusion systems, and liposomes. Formulations for inhalation may contain excipients, for example, lactose, or may be aqueous solutions containing, for example, polyoxyethylene-9-lauryl ether, glycocholate and deoxycholate, or may be oily solutions for administration in the form of nasal drops, or as a gel.

The following examples are to illustrate the invention. They are not meant to limit the invention in any way. Assays for compounds that inhibit cell death, as described below, can be performed with numerous variations, which will be described after the examples.

### EXAMPLE I

### Use of Superior Cervical Ganglia Neurons to Test Compounds for Their Effect on Neuronal Cell Death

Primary sympathetic neurons of the superior cervical ganglia are cultured in 96-well tissue culture plates by standard methods. NGF is withdrawn from the media to induce cell death and concomitantly, compounds to be tested are added to the cells in a range of concentrations. At appropriate timepoints, e.g., between 0 and 36 hours, the treated samples are lysed by standard techniques and the cell lysates are subjected to the appropriate assay as described below.

### EXAMPLE II

### ELISA for the detection of compounds that inhibit sympathetic neuronal cell death

Enzyme-linked immunosorbant assays (ELISAs) are easily incorporated into high-throughput screens designed to test large numbers of compounds for their ability to modulate levels of a given protein. When used in the methods of the invention, changes in a given protein level of a sample, relative to a control, reflect changes in the apoptotic status of the cells within the sample. Protocols for ELISA may be found, for example, in Ausubel et al., *Current* Protocols *in Molecular Biology,* John Wiley & Sons, New York, NY, 1997. Lysates from neuronal cells treated with potential cell death modulators are prepared (see, for example, Ausubel et al., *supra),* and are loaded onto the wells of microtiter plates coated with "capture" antibodies against one of the death markers (e.g., p53, p21, p27, and pRb), antibodies specific for p53, p21, p27, and pRb polypeptides being available, for example, from commercial sources such as CalBiochem, Santa Cruz Biotechnology, and Transduction Laboratories. Unbound antigen is washed out, and a death marker-specific antibody, coupled to an agent to allow for detection, is added. Agents allowing detection include alkaline phosphatase (which can be detected following addition of colorimetric substrates such as *p*-nitrophenylphosphate), horseradish peroxidase (which can be detected by chemiluminescent substrates such as ECL, commercially available from Amersham) or fluorescent compounds, such as FITC (which can be detected by fluorescence polarization or time-resolved fluorescence). The amount of antibody binding, and hence the level of a death marker within a lysate sample, is easily quantitated on a microtiter plate reader. In the case of the pRb death marker, wherein absolute levels of pRb do not change in dying cells, rather, the amount of hyperphosphorylated pRb increases, duplicate ELISAs are employed. One ELISA measures total pRb, as in the aforedescribed assay, and the second ELISA measures phosphorylated pRb. In the second ELISA, the wells of the microtiter plate are coated with the same anti-pRb antibody as in the first ELISA, which captures all of the pRb within the neuronal lysate applied to the coated well. However, in the second pRB ELISA, after washing away unbound protein, a secondary antibody specific for phosphotyrosine is applied. Addition of the anti-phopshotyrosine antibody allows the quantitation of phosphorylated pRb. Anti-phosphotyrosine antibodies are available, for example, from commercial sources such as Upstate Biotechnology and Transduction Laboratories.

As a baseline control for death marker levels in non-dying cells, a sample that is continuously exposed to NGF is included. As a baseline control for death marker levels in dying cells, a sample in which NGF is withdrawn and not replaced is included. MAP kinases and the p85 subunit of PI3 kinase are used as internal standards for absolute protein levels, since their levels do not change over the preferred timecourse (0 to 36 hours after NGF withdrawal). A positive assay result, for example, identification of a compound that decreases neuronal apoptosis, is indicated by a decrease in death marker levels, relative to the death marker level observed in cells which are induced to die without rescue. Death marker levels in neurons treated with a death inhibitory compound are modulated, relative to death marker levels in dying neurons, as displayed in Table I.

### EXAMPLE III

### Reporter gene assays for compounds that inhibit neuronal cell death

Assays employing the detection of reporter gene products are extremely sensitive and readily amenable to automation, hence making them ideal for the design of high-throughput screens. Assays for reporter genes may employ, for example, colorimetric, chemiluminescent, or fluorometric detection of reporter gene products. Many varieties of plasmid and viral vectors containing reporter gene cassettes are easily obtained. Such vectors contain cassettes encoding reporter genes such as lacZ/β-galactosidase, green fluorescent protein, and luciferase, among others. Cloned DNA fragments encoding transcriptional control regions of interest are easily inserted, by DNA subcloning, into such reporter vectors, thereby placing a vector-encoded reporter gene under the transcriptional control of any gene promoter of interest. The transcriptional activity of a promoter operatively linked to a reporter gene can then be directly observed and quantitated as a function of reporter gene activity in a reporter gene assay.

### Reporter gene assay of primary sympathetic neurons from transgenic mice

Primary neurons from mice containing one or more reporter transgene constructs are cultured, cell death is induced, and compounds to be tested for their death-modulating activity are added to the neurons (e.g., as in Example I). At appropriate timepoints, cells are lysed and subjected to the appropriate reporter assays, for example, a colorimetric or chemiluminescent enzymatic assay for lacZ/β-galactosidase activity, or fluorescent detection of GFP. Changes in reporter gene activity of samples treated with test compounds, relative to reporter gene activity of appropriate control samples as suggested in Example II, indicate the presence of a compound that modulates neuronal cell death.

In one embodiment, one transgene could comprise a reporter gene such as lacZ or green fluorescent protein (GFP), operatively linked to a promoter from a death gene such as those encoding p53,p21, p27, Transgenes may be present within the genomic DNA of a neuron to be tested, or may be transiently introduced into a neuron. A second transgene, comprising a second reporter gene operatively linked to a second promoter, is included as an internal control. This could be a reporter gene operatively linked, for example, to the neuron-specific Tα1 α-tubulin gene (see, e.g., US-A-5 661 032. The Tα1 α-tubulin gene is abundantly expressed in developing neurons during morphological growth, and also is abundantly expressed in mature neurons during the process of target re-innervation. Hence, the amount of activity resulting from a reporter gene that is operatively linked to the Tα1 α-tubulin promoter will indicate the proportion of live neurons within a sample, relative to the appropriate controls. The range of changes in death marker-reporter gene product levels resulting from changes in neuronal cell death levels is proportional to the range of changes in death marker protein seen in Example II.

### Reporter gene assay in adenovirus-transduced primary sympathetic neurons

Primary neurons from transgenic or non-transgenic animals are isolated and infected with an adenovirus containing a reporter gene construct of interest, such as those described immediately above. The neurons are treated with test compounds and apoptosis is initiated, and reporter activity is measured and interpreted as provided herein.

Alternatively, a gene whose expression modulates neuronal cell death can be introduced by adenovirus-mediated gene transfer. For example, death markers (e.g., p53, p21, p27, and Rb) can be introduced into neurons by adenovirus-mediated gene transfer. Increased expression resulting from this gene transfer induces cells to die, and in this manner, test compounds that specifically interfere with death marker-mediated neuronal cell death can be isolated. In this case, a compound that modulates neuronal death is defined as one that modulates endogenous (i.e., encoded by neuronal genomic DNA, not adenovirus vector-encoded)death marker levels, or alternatively, a genomic or adenovirally-introduced reporter gene can be used as an indicator for modulation of cell death, as described in Example III.

Adenovirus mediated gene transfer is performed according to standard techniques, such as those described in Slack, R. S., et al, *J. Cell Biol*., VoL 135:1085, 1996.

### EXAMPLE IV

### Quantitative PCR of death marker mRNA as an assay for compounds that inhibit sympathetic neuronal cell death

The polymerase chain reaction (PCR), when coupled to a preceding reverse transcription step (rtPCR), is a commonly used method for detecting vanishingly small quantities of a target mRNA. When performed within the linear range, with an appropriate internal control target (employing, for example, a housekeeping gene such as actin), such quantitative PCR provides an extremely precise and sensitive means of detecting slight modulations in mRNA levels. Moreover, this assay is easily performed in a 96-well format, and hence is easily incorporated into a high-throughput screening assay. Neurons are cultured, treated with test compounds, and induced to die as described in the preceding examples. The neurons are then lysed, the mRNA is reverse-transcribed, and the PCR is performed according to commonly used methods, (such as those described in Ausubel et al., *Current Protocols in Molecular Biology,* John Wiley & Sons, New York, NY, 1997), using oligonucleotide primers that specifically hybridize with the nucleic acid of interest In one embodiment, the target mRNA could be that of one or more of the death markers, e.g., p53, p21, p27. Analogously to the death marker polypeptide result described in Example II, changes in product levels of samples exposed to test compounds, relative to control samples, indicate test compounds with neuronal death-modulating activity.

DNA sequences that are used to make oligonucleotide primers for use in death marker rtPCR assays are found in the GenBank database, according to the accession numbers listed below, and in the references listed below.

### p53

*mus musculus* Genbank Accession No. X01237 K01700
Zakut-Houri et al., Nature 306:534 (1983)
Bienz et al., Embo J. 3:2174 (1984)

### Bcl2

*Homo sapiens* Genbank Accession No. 2144603
*Rattus norvegicus* Genbank Accession No. 1705443
*Mus musculus* Genbank Accession No. M16506
*Homo sapiens* (Bcl2 β) Genbank Accession No. 68975
*Mus muculus* (Bcl2 α) Genbank Accession No. 231633
*Homo sapiens* (Bcl2 α) Genbank Accession No. M13994
Tsujimoto et al., PNAS 83:5214 (1986) (human)
Negrimi et al., Cell 48:455 (1987) (mouse-α and β)
Eguchi et al., Nucleic Acid Res. 20:4187 (1992) (mouse-α)
Sato et al., Gene 140:291 (1989) (rat-α)
Tanaka et al., Blood 79:229 (1992) (rat-α)
Cleary et al., Cell 47:19 (1986) (human-α)
Seto, Embo J. 7:123 (1983) (human-α)
Hua et al., Oncogenes Res. 2:263 (1988) (human)

### Bax

*Rattus norvegicus* Genbank Accession No. 2143610
*Mus musculus* Genbank Accession No. 728946
*Mus musculus* Genbank Accession No. L22472
Tilly et al., Endocrinology 136:232 (1995) (rat)
Oltvai et al., Cell 74:609 (1993) (mouse)

### p27

*Rattus norvegicus* Genbank Accession No. D83792
*Homo sapiens* Genbank Accession No. U10906
*Mus musculus* Genbank Accession No. 1168872
*Mus musculus* Genbank Accession No. U09968 Toyoshima, Cell 78:67 (1994) (mouse)
Polyak, Cell 78:59 (1994) (mouse)
Nomura et al., Gene 191:211 (1997) (rat)

### p21

*Mus musculus* Genbank Accession No. U24173
Deiry et al., Cancer Res 55:2910 (1995) (mouse)

### Bad

*Mus musculus* Genbank Accession No. L37296
*Mus musculus* Genbank Accession No. 639779
Yang et al., Cell 80:285 (1995) (mouse)

### EXAMPLE V

### Demonstration that NGF withdrawal induces apoptosis in primary sympathetic neurons

To confirm that primary sympathetic neurons die by apoptotic cell death, neurons were infected with a recombinant adenovirus expressing E1B55K, an adenoviral protein known to inhibit apoptosis by inhibiting p53. After adenovirus infection, NGF was withdrawn, and neuronal survival was then quantitated by MTT assay, as in Slack et al. *supra.* E1B55K inhibits death in NGF-deprived neurons (Figure 1), confirming that NGF-induced death is an apoptotic process involving p53. Bcl2, a protein that can inhibit apoptosis, also inhibits cell death in NGF-deprived neurons. These results confirmed that the NGF withdrawal assay could be used to detect proteins which undergo alterations as part of the commitment to apoptosis of neurons.

### Other Embodiments

While the invention has been described in connection with specific embodiments thereof, it will be understood that it is capable of modifications and variations within the scope of the appended claims.

## Claims

1. A method of identifying a compound which modulates neuronal apoptosis, said method comprising the steps of:
(a) exposing a neuron to a test compound;
(b) exposing said neuron to a stimulus which is capable of initiating apoptosis of a neuronal cell; and
(c) assaying for an alteration in the level of p53, p21, p27, or phosphorylated Rb polypeptides or in the level of p53, p21 or p27 gene expression relative to a neuron not exposed to said test compound, a decrease in said level indicating a compound which reduces neuronal apoptosis, and an increase in said level indicating a compound which enhances neuronal apoptosis;
with proviso that the method is not performed on the human body.

2. The method of claim 1, wherein said decrease is at least a 30% decrease in the level of p53, a 30% decrease in the level of p21 or a 30% decrease in the level of p27.

3. The method of claim 1, wherein said stimulus is selected from the group consisting of serum withdrawal, growth factor withdrawal, staurosporine exposure, glutamine exposure, NMDA exposure, DNA damage, exposure to reactive oxygen species, exposure to physical trauma, and axatomy.

4. The method of claim 3, wherein said growth factor withdrawal is neurotrophin withdrawal.

5. The method of claim 1, wherein said neuron further comprises a reporter gene operably linked to a transcriptional control region from a gene encoding p53, p21, or p27.

6. The method of claim 5, wherein said assay further comprises testing for an altered level of reporter gene product.

7. The method of claim 1, wherein said assaying measures the expression levels of at least two proteins.

8. The method of claim 1, wherein said neuron is selected from the group consisting of a sympathetic neuron, a cortical neuron, a motor neuron, and a hippocampal neuron.

## Patentansprüche

1. Ein Verfahren zur Identifizierung einer Verbindung, die neuronale Apoptose moduliert, umfassend die Schritte:
(a) In Kontakt bringen eines Neurons mit einer Testverbindung
(b) In Kontakt bringen des Neurons mit einem Reiz, der die Initiierung von Apoptose einer neuronalen Zelle auslösen kann; und
(c) Untersuchung hinsichtlich einer Veränderung des Spiegels von p53, p21, p27 oder von phosphorylierten Rb-Polypeptiden oder des Spiegels an Genexpression von p53, p21 oder p27 relativ zu einem Neuron, das nicht mit der Testverbindung in Kontakt gebracht wurde, wobei eine Abnahme des Spiegels auf eine Verbindung hinweist, die neuronale Apoptose vermindert und wobei eine Steigerung des Spiegels auf eine Verbindung hinweist, die neuronale Apoptose steigert;
unter der Voraussetzung, dass das Verfahren nicht am menschlichen Körper durchgeführt wird.

2. Verfahren gemäß Anspruch 1, wobei die Abnahme des Spiegels von p53 jeweils eine Abnahme um wenigstens 30 % des Spiegels von p53, p21 oder p27 ist.

3. Verfahren gemäß Anspruch 1, wobei der Reiz ausgewählt ist aus der Gruppe bestehend aus Serumentzug, Wachstumsfaktorentzug, in Kontakt bringen mit Staurosporin, in Kontakt bringen mit Glutamin, in Kontakt bringen mit NMDA, DNA-Schädigung, in Kontakt bringen mit reaktiven Sauerstoffspezies, in Kontakt bringen mit physischem Trauma und Axatomie.

4. Verfahren gemäß Anspruch 3, wobei der Wachstumsfaktorentzug ein Entzug von Neurotrophin ist.

5. Verfahren gemäß Anspruch 1, wobei das Neuron weiterhin ein Reportergen umfasst, das funktionell an einen Transkriptions-Kontrollbereich eines Gens, das für p53, p21 oder p27 kodiert, gekoppelt ist.

6. Verfahren gemäß Anspruch 5, wobei die Untersuchung weiterhin das Testen hinsichtlich eines veränderten Spiegels des Reporter-Genprodukts umfasst.

7. Verfahren gemäß Anspruch 1, wobei die Untersuchung die Expressionsspiegel von wenigstens zwei Proteinen misst.

8. Verfahren gemäß Anspruch 1, wobei das Neuron ausgewählt ist aus der Gruppe bestehend aus einem sympathischen Neuron, einem kortikalen Neuron, einem motorischen Neuron und einem Neuron des Hippocampus.

## Revendications

1. Procédé permettant d'identifier un composé qui module l'apoptose neuronale, ledit procédé comprenant les étapes de:
(a) exposition d'un neurone à un composé-test;
(b) exposition du dit neurone à un stimulus qui est capable d'initier l'apoptose d'une cellule neuronale; et
(c) détermination d'une altération du niveau de polypeptides p53, p21, p27 ou Rb phosphorylé ou du niveau d'expression de gène p53, p21 ou p27 par rapport à un neurone non-exposé au dit composé-test, une diminution du dit niveau indiquant un composé qui réduit l'apoptose neuronale, et une augmentation du dit niveau indiquant un composé qui renforce l'apoptose neuronale;
avec pour condition que le procédé n'est pas mis en oeuvre sur le corps humain.

2. Procédé selon la revendication 1, dans lequel ladite diminution est au moins une diminution de 30% du niveau de p53, une diminution de 30% du niveau de p21 ou une diminution de 30% du niveau de p27.

3. Procédé selon la revendication 1, dans lequel ledit stimulus est choisi dans le groupe consistant en élimination de sérum, élimination de facteur de croissance, exposition à la staurosporine, exposition à la glutamine, exposition au NMDA, endommagement d'ADN, exposition à une espèce oxygénée réactive, exposition à un traumatisme physique et axatomie.

4. Procédé selon la revendication 3, dans lequel ladite élimination de facteur de croissance est une élimination de neurotrophine.

5. Procédé selon la revendication 1, dans lequel ledit neurone comprend en outre un gène reporter lié opérationnellement à une région de contrôle transcriptionnel provenant d'un gène codant pour p53, p21 ou p27.

6. Procédé selon la revendication 5, dans lequel ladite détermination comprend en outre le test d'un niveau altéré de produit de gène reporter.

7. Procédé selon la revendication 1, dans lequel ladite détermination mesure les niveaux d'expression d'au moins deux protéines.

8. Procédé selon la revendication 1, dans lequel ledit neurone est choisi dans le groupe consistant en un neurone sympathique, un neurone cortical, un neurone moteur et un neurone de l'hippocampe.
